# EUROPÄISCHE PATENTSCHRIFT

(11) **EP 0 898 464 B1**
(45) Veröffentlichungstag und Bekanntmachung des Hinweises auf die Patenterteilung: **27.06.2001**
(21) Anmeldenummer: 97919237.4
(22) Anmeldetag: 02.05.1997
(51) Int. Cl.: A61B 17/17

(54) **ZIELGERÄT ZUR VERRIEGELUNG DER FUSSPARTIE VON MARKNÄGELN**
TARGETING APPARATUS FOR LOCKING THE BASE PART OF INTRAMEDULLARY NAILS
APPAREIL-CIBLE POUR BLOQUER LA PARTIE DE BASE DE CLOUS D'OSTEOSYNTHESE INTRAMEDULLAIRES

(30) Priorität: 04.05.1996 DE 29608071 U
(43) Veröffentlichungstag der Anmeldung: 03.03.1999
(73) Patentinhaber: Synthes AG, Chur, 7002 Chur (CH)
(72) Erfinder: KRETTEK, Christian, D-30629 Hannover (DE); KÖNEMANN, Bernd, D-29223 Celle (DE); PERRIER, Alexandre, CH-4053 Basel (CH); SENN, Peter, CH-4437 Waldenburg (CH)
(74) Vertreter: Lusuardi, Werther Giovanni, Dr.
(86) Internationale Anmeldenummer: CH9700172
(87) Internationale Veröffentlichungsnummer: WO9741781

(56) Entgegenhaltungen:
- WO-A-92/01422
- WO-A-96/03085
- CH-A- 668 692
- DE-A- 4 306 724

## Beschreibung

Die Erfindung bezieht sich auf ein Zielgerät zum Positionieren und Einbringen von osteosynthetischen Befestigungselementen, insbesondere von Verriegelungs-Schrauben oder Bolzen in die Fusspartie eines Marknagels gemäss dem Oberbegriff des Patentanspruchs 1.

Ein erfolgreiches Plazieren der Verriegelungsbolzen in die Fusspartie eines Verriegelungsmarknagels ist ein schwieriges und zeitaufwendiges Unterfangen. Das am Markt meist verwendete Verfahren ist die Freihandmethode mit Hilfe eines röntgendurchlässigen Winkelgetriebes. Die Genauigkeit dieses Verfahrens hängt von der Geschicklichkeit des Operateurs ab und schliesst den Nachteil einer zumeist hohen Strahlenbelastung mit ein.

Ein Verzicht auf die Verwendung einer Röntgenvorrichtung streben beispielsweise die Erfindungen der Zielvorrichtungen zur distalen Verriegelung von Marknägeln aus der DE-A1 4306724 und DE-A1 4414177 an. Die Treffsicherheit dieser Zielvorrichtungen hängt aber davon ab, wie sehr der in den Knochen eingebrachte Marknagel noch seine ursprünglichen Form vor der Insertion beibehält. Diese Zielgeräte tolerieren weder implantationsbedingte Verbiegung noch Verwindung des Marknagels zwischen seiner Kopfpartie und seiner Fusspartie. Eine vom Erfinder vorgenommene Untersuchung über die implantationsbedingte Nagelverformung von soliden Marknägeln (SYNTHES, UTN) zeigt aber auf, dass diese Grundvoraussetzung für die Treffsicherheit nicht gegeben ist; die Verwindung der soliden Nägel ist gering und in der Praxis vernachlässigbar, die Verbiegung ist jedoch wesentlich. Auf Grund der Verbiegung der Marknägel sind die oben erwähnten Zielvorrichtungen nicht treffsicher. Die Resultate dieser Untersuchung wurden in der Zeitschrift "Der Unfallchirurg" im September 1996 unter dem Titel "Analyse implantationsbedingter Nagelverformung und röntgenmorphometrische Untersuchungen als Grundlage für ein Zielgerät zur distalen Verriegelung ohne Röntgenbildverstärker" veröffentlicht.

Eine weitere bekannte Zielvorrichtung (CH-A5 668692) hat sich die Aufgabe gestellt, zu ermöglichen, dass die Achse des bzw. jedes zum Ausrichten auf ein Loch des distalen Nagel-Endabschnitts bestimmten Lochs der Zielvorrichtung auch dann tatsächlich genau zum Fluchten mit der Achse eines im distalen Endabschnitt eines Nagels vorhandenen Lochs gebracht werden kann, wenn der Nagel zwischen seinem proximalen Ende und den Löchern seines distalen Endabschnitts gebogen und/oder verwunden ist. Diese Zielvorrichtung bedarf jedoch wiederum einer Röntgenvorrichtung. Sie reduziert die Strahlenbelastung zwar, kann aber nicht darauf verzichten.

Aus der US 5,281,224 FACCIOLI ET AL. und US 5,433,720 FACCIOLI ET AL. ist eine Zielvorrichtung bekannt, welche den Verzicht auf eine Röntgenvorrichtung anstrebt und zugleich eine Nagelverformung bei der Insertion toleriert. Diese Zielvorrichtungen basieren auf der Idee einer Nachjustierung der Achsen der Löcher der Bohrlehre auf die Flucht der Achsen der Verriegelungslöcher im distalen Endabschnitt des Nagels mit Hilfe eines Metall-Detektors. Dieser Metall-Detektor funktioniert über Elektronik und die Herstellung eines magnetischen Feldes. Unterstützt wurden diese Erfindungen durch die Firma ORTHOFIX S.R.L. Auf dem Markt sind dieses Zielgeräte bisher aber nicht bekannt.

Schliesslich hat die Firma ORTHOFIX S.R.L. ein Zielgerät auf den Markt gebracht, bei welchem mittels eines dornförmigen Abstandgebers ein sogenannter "Direktkontakt" auf die anteriore Vorderkante des distalen Endabschnitts des Marknagels herstellbar ist. Das Dokument WO-A 9603085 beschreibt dieses Zielgerät. Dieses Zielgerät erhebt den Anspruch, gänzlich auf die Kontrolle mittels einer Röntgenvorrichtung verzichten zu können. Es toleriert eine Verbiegung des Nagels in der antero-posterioren Ebene. In der mediolateralen Ebene ist die tolerierbare Verbiegung des Nagels jedoch reduziert auf die Kontaktfläche des Stabilisierungsdorns. Diese ist relativ klein. Zudem ist dieses Zielgerät so konzipiert, dass distale Verriegelungsbolzen nur mit einer medio-lateralen Ausrichtung eingebracht werden können. Eine Marknagelverriegelung in einer einzigen Ebene ist aber unvollständig. Zur Verbesserung der Kippstabilität von Marknägeln ist eine Verriegelung in mehr als einer radialen Ebene relativ zum Marknagel anzustreben.

Hier will die Erfindung Abhilfe schaffen. Es ist primäres Ziel der vorliegenden Erfindung, eine Zielvorrichtung zur Verriegelung der Fusspartie von Marknägeln zu schaffen, welche - ohne die Verwendung einer Röntgenvorrichtung - das exakte Plazieren von Verriegelungsbolzen in mehr als einer radialen Ebene relativ zum Marknagel erlaubt und dies auch, wenn der Marknagel bei der Implantation einer wesentlichen Verbiegung unterliegt, wie dies der Realität entspricht.

Die Erfindung löst die gestellte Aufgabe mit einem Zielgerät, welches die Merkmale des Anspruchs 1 aufweist.

Weitere vorteilhafte Ausgestaltungen der Erfindung sind in den abhängigen Ansprüchen gekennzeichnet. Die Erfindung und Weiterbildungen der Erfindung werden im folgenden anhand der teilweise schematischen Darstellung eines Ausführungsbeispiels an einer rechten Tibia noch näher erläutert.

Es zeigen:
Fig. 1 eine perspektivische Ansicht des erfindungsgemässen an einen tibialen Marknagel gekuppelten Zielgerätes mit einer ersten Abstandgebereinheit und einer zweiten Abstandgebereinheit;
Fig. 2 eine Ansicht der ersten Abstandgebereinheit des erfindungsgemässen Zielgerätes in der intraoperativen Anwendung;
Fig. 3 eine mögliche Ausführungsvariante des Abstandgebers der ersten Abstandgebereinheit; und
Fig. 4 eine Ansicht der zweiten Abstandgebereinheit des erfindungsgemässen Zielgerätes in der intraoperativen Anwendung.

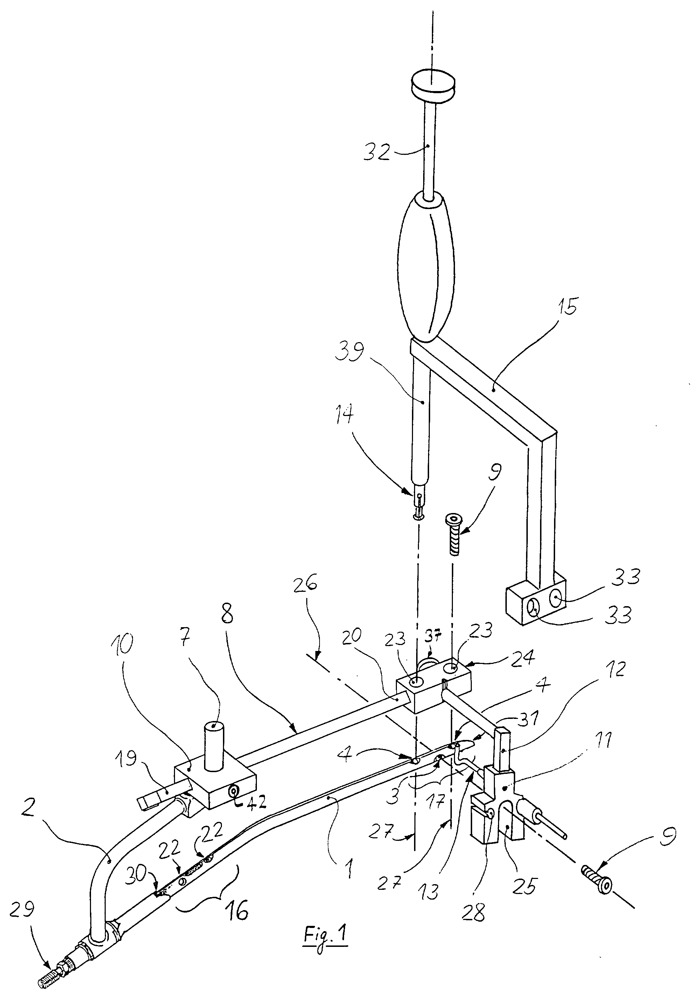

Das in Fig. 1 dargestellte Zielgerät besteht im wesentlichen aus einer longitudinalen Zielschiene 8 mit einem ersten Abschnitt 19 und einem zweiten Abschnitt 20. Der erste Abschnitt 19 ist als Prismenführung ausgebildet und kann mittels eines Kupplungsmittels 10 - in Form eines Gelenkkörpers - über einem handelsüblichen U-förmigen Marknagel-Zielbügel 2 am Kopfende 30 des Marknagels 1 lösbar befestigt werden. Die Zielschiene 8 kann innerhalb des Kupplungsmittels 10 longitudinal verschoben werden, um die stufenlose Einstellung auf eine gewählte Nagellänge zu gestatten. Das Kupplungsmittel 10 besteht aus einer als Drehgelenk dienenden Gelenkachse 7, welche in die Führungsbohrungen des U-förmigen Zielbügels 2 einführbar ist. Der Zielbügel 2 lässt sich in üblicher Weise mittels Schraubenverschluss 29 am Kopfende 30 des Marknagels 1 aufsetzen.

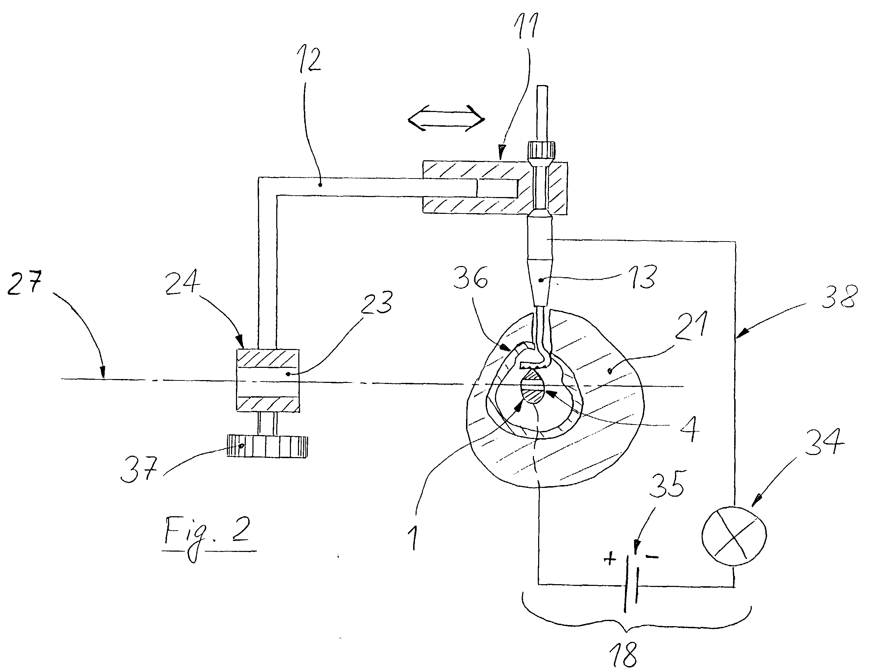

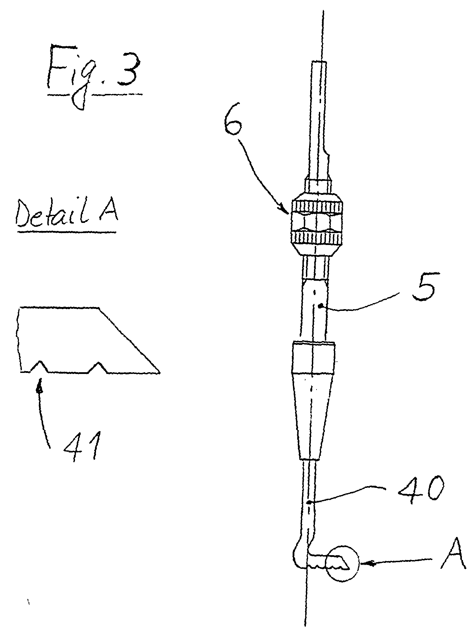

Am zweiten Abschnitt 20 der Zielschiene 8 ist eine Bohrlehre 24 angeordnet, welche zwei quer zur Zielschiene 8 verlaufende, durchgehende, mediolaterale Führungsbohrungen 23 zum Positionieren und Einbringen mediolateraler osteosynthetischer Befestigungselemente 9 in Form von Verriegelungs-Schrauben aufweist. Die Bohrlehre 24 ist weiter mit einer ersten Abstandgebereinheit 11,12,13 lösbar verbunden. Diese besteht aus einem bogenförmigen Abstandgeber-Bügel 12, der an seinem der Bohrlehre 24 abgewandten Ende einen Schlitten 11 aufweist. Dieser Schlitten 11 ist mit einem anteroposterioren Führungsschlitz 25 versehen. Der Schlitten 11 ist im weiteren mit zwei Führungskanälen 28 versehen, welche zum Positionieren und zur Aufnahme des anterioren Abstandgebers 13 dienen. In Fig. 1 wird der distal gelegene Führungskanal 28 dazu benutzt; es funktioniert jedoch ebenfalls mit dem proximal gelegenen Führungskanal 28, ganz nach Belieben des Operateurs. Die Details des anterioren Abstandgebers 13 werden in der Detailbeschreibung Fig. 2 und Fig. 3 berücksichtigt.

Das Kupplungsmittel 10 am ersten Abschnitt 19 der Zielschiene 8 ist derart ausgebildet, dass eine Abwinkelung der Zielschiene 8 relativ zu dem daran gekuppelten Marknagel 1 möglich ist.

Die Abwinkelung erfolgt dabei um eine im wesentlichen parallel zur zentralen Achse 27 des mediolateralen Verriegelungsloches 4 verlaufenden Gelenkachse 7, vorzugsweise in Form eines Drehgelenkes.

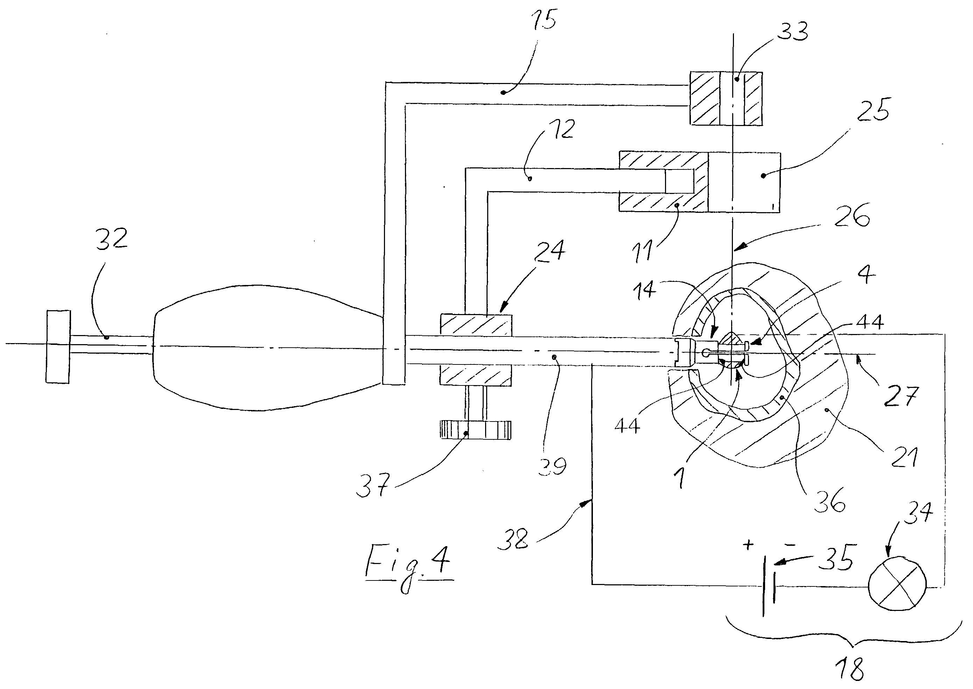

Die zweite Abstandgebereinheit 14,15 umfasst einen Zielbügel 15 und einen Kontaktgeber. Der Zielbügel 15 ist einerseits mit zwei anteroposterioren Führungsbohrungen 33 ausgestattet, die zum Positionieren und Einbringen eines Befestigungselementes 9 in das anteroposteriore Verriegelungsloch 3 der Fusspartie 17 des Marknagels 1 dienen. Im hiesigen Ausführungsbeispiel an einer rechten Tibia wird dazu die distal gelegene Führungsbohrung 33 benutzt. Die hier proximal gelegene Führungsbohrung 33 würde bei einer identischen Anwendung des Zielgerätes an einer linken Tibia distal zu liegen kommen und für diesen Fall benutzt werden. Andererseits ist der Zielbügel 15 mit einem Kontaktgeber 14 versehen, der auf ein mediolaterales Verriegelungsloch 4 in der Fusspartie 17 des Marknagels 1 Bezug nimmt. Der Kontaktgeber 14 kann eine Kontaktverbindung mit dem Verriegelungsloch 4 eingehen, so dass eine Kontrolle der Kontaktverbindung ohne direkte Ansicht oder Röntgenkontrolle möglich ist. Dazu sind mehrere Ausführungsarten möglich, die im Detail bei der Beschreibung der Fig. 4 berücksichtigt werden.

In Fig. 2 ist ein Schnitt durch einen Unterschenkel 21 dargestellt. Der quer zur Zielschiene 8 verschiebbare Schlitten 11 und die in L-, T- oder Schneidenform gestaltete Auflagefläche des anterioren Abstandgebers 13 erlauben anterioren Nagelkontakt auch wenn sich dieser um mehrere Millimeter verschoben hat. Der Kontakt zwischen anteriorem Abstandgeber 13 und Marknagel 1 kann ohne den Einsatz einer Röntgenvorrichtung folgendermassen kontrolliert werden:
- durch metallische Kratzgeräusche der Verzahnung der Auflagefläche des Abstandgebers 13 auf der Oberfläche des Marknagels 1; oder
- durch die visuelle oder sonore Rückkoppelung (Kontaktinformation) einer Kontaktanzeigevorrichtung 18, die über Elektrizitätsfluss funktioniert. Die elektrische Kontaktanzeigevorrichtung 18 besteht im wesentlichen aus einer Energiequelle 35 (Batterie), aus einem Signalgeber 34 und aus einer elektrischen Leitung 38, welche den anterioren Abstandgeber 13 und den Marknagel 1 miteinander verbindet. Wird nun der Stromkreis durch Kontakt zwischen Abstandgeber 13 und Marknagel 1 geschlossen, erhält der Operateur über den Signalgeber 34 eine Rückkoppelung (Kontaktinformation). Sobald Kontakt zwischen dem anterioren Abstandgeber 13 und dem Marknagel 1 besteht, fluchten die mediolateralen Führungsbohrungen 23 der Bohrlehre 24 mit den mediolateralen Verriegelungslöchern 4 des Marknagels 1.

Fig. 3 zeigt eine bevorzugte Ausführungsvariante des anterioren Abstandgebers 13. Der L-förmige Haken 40 toleriert durch die Auflagefläche der Unterseite des kurzen L-Schenkels auf den Marknagel 1 eine implantationsbedingte Verbiegung des Marknagels 1 im Bereich der Länge dieses kurzen L-Schenkels.

Ebenfalls wird durch die L-Form des Abstandgebers 13 ein gewisser Freiraum für die Wahl der Position der Kontakteröffnung in den Knochen eingeräumt (siehe Schlitten 11 auf dem Abstandgeberbügel 12). Durch die Kerben 41 auf der Unterseite des kurzen L-Schenkels des L-Hakens 40 können metallische Kratzgeräusche auf der Oberfläche des Marknagels 1 erzeugt werden. Diese Geräusche dienen dem Operateur als Rückkoppelung, dass der Abstandgeber 13 auf dem Marknagel 1 aufsteht. Durch die Auswechselbarkeit des Abstandgebers 13 und der variablen Länge der L-, T- oder schneidenförmigen Haken 40 in longitudinaler Richtung des Abstandgebers 13 wird eine präoperative Kalibrierung der Länge zwischen dem L-, T-, oder schneidenförmigen Haken 40 und dem Schlitten 11 auf den gewählten Durchmesser des Marknagels ermöglicht. Die Verbindungsmutter 6 ermöglicht eine stabile Verbindung des Abstandgebers 13 mit dem an die Zielschiene 8 gekoppelten Abstandgeberbügel 12 mit Schlitten 11. Die Verbindung findet durch Einführen des Schaftes 5 des Abstandgebers 13 im Führungskanal 28 und durch Festziehen der Verbindungsmutter 6 statt.

In Fig. 4 ist ein Schnitt durch einen Unterschenkel 21 dargestellt, wobei der anteroposteriore Zielbügel 15 durch einen mittels Spreizdorn 32 verspreizbaren Kontaktgeber 14 mit einem der beiden mediolateralen Verriegelungslöcher 4 des Marknagels 1 verbunden ist. Für diese Verbindung sind mehrere Ausführungsarten möglich:
- Der Kontaktgeber 14 weist ein verspreizbares, geschlitztes Ende auf, welches in das Verriegelungsloch 4 des Marknagels 1 einführbar ist. Dadurch erfolgt ein korrektes Festsitzen durch die Abstandgeberflächen 44 mit Kontakt auf der medialen Seite des Verriegelungsloches 4 einerseits und auf der lateralen Seite des Verriegelungsloches 4 anderseits. Diese Ausführung wird in Fig. 4 dargestellt;
- Die Befestigung des Kontaktgebers 14 erfolgt über das Festziehen und Verklemmen von zwei ineinander geschobenen Abstandgebern, einer mit Kontakt auf der medialen Seite des Verriegelungsloches 4, der andere mit Kontakt auf der lateralen Seite des Verriegelungsloches 4;
- Durch Aufsitzen einer Kontaktfläche 44 nur einseitig des Verriegelungsloches 4 und sonore Rückkoppelung (Kontaktinformation) über Aufschlaggeräusche;
- Durch Aufsitzen einer Kontaktfläche 44 nur einseitig des Verriegelungsloches 4 und visuelle oder sonore Rückkoppelung (Kontaktinformation) einer Kontaktanzeigevorrichtung 18, die über Elektrizitätsfluss funktioniert. Die elektrische Kontaktanzeigevorrichtung 18 besteht im wesentlichen aus einer Energiequelle 35 (Batterie), aus einem Signalgeber 34 und aus einer elektrischen Leitung 38, welche den Kontaktgeber 14 und den Marknagel 1 miteinander verbindet. Wird nun der Stromkreis durch Kontakt zwischen Kontaktgeber 14 und Marknagel 1 geschlossen, erhält der Operateur über den Signalgeber 34 eine Rückkoppelung (Kontaktinformation).

Sobald die Kontaktverbindung zwischen dem Kontaktgeber 14 und dem Marknagel 1 besteht, kann die anteroposteriore Führungsbohrung 33 des Zielbügels 15 über den Führungsschlitz 25 des Abstandgeberbügels 12 geschwenkt werden, so dass eine der anteroposterioren Führungsbohrungen 33 mit dem Führungsschlitz 25 fluchtet. In dieser Position fluchtet diese Führungsbohrung 33 ebenfalls mit dem anteroposterioren Verriegelungsloch 4 des Marknagels 1. Dabei kann immer nur eine der Führungsbohrungen 33 in die Flucht mit dem Führungsschlitz 25 gebracht werden; die andere Führungsbohrung 33 ist für die identische Anwendung des Zielgerätes an einer contralateralen Tibia bestimmt.

Im folgenden wird nun kurz die Operationstechnik der Verriegelung der Fusspartie eines Marknagels mit dem erfindungsgemässen Zielgerät am Beispiel eines tibialen Marknagels erläutert. Das Verfahren besteht im wesentlichen aus drei Positionierungsschritten:

### A) Justierung des Zielschiene (8) in der kraniokaudalen Ebene auf die Position der Verriegelungslöcher 3,4

Auf den noch nicht implantierten Marknagel 1 wird an seinem Kopfende 30 ein üblicher, beispielsweise aus der CH-A5 668.692 bekannter U-förmiger Marknagel-Zielbügel 2 montiert, mit welchem proximale Verriegelungsschrauben 9 in den dazu vorgesehenen Verriegelungslöchern 22 des Marknagels 1 gesetzt werden können. Nun wird die Zielschiene 8 für das Positionieren und Einbringen der Schrauben 9 in die distalen mediolateralen Verriegelungslöcher 4 an das freie Ende des proximalen Zielbügels 2 gekoppelt. Dies geschieht durch Einführen einer als Drehgelenk dienenden Gelenkachse 7 in die Führungsbohrung des proximalen Zielbügels 2. Die Zielschiene 8 wird nun auf die Länge des verwendeten Marknagels 1 justiert. Dies geschieht durch Schieben der Zielschiene 8 im Kupplungsmittel bei gelöster Klemmschraube 42 bis die Führungsbohrungen 23 mit den Verriegelungslöchern 4 fluchten.
Anschliessend wird der Marknagel 1 in die Tibia eingeführt, wobei er sich in der Regel verbiegt, so das eine Justierung des Zielgerätes in anteroposteriorer und mediolateraler Ebene erforderlich ist.

### B) Justierung der Zielschiene 8 in der anteroposterioren Ebene auf die Position der Verriegelungslöcher 4

Die anteriore Kante der Fusspartie 17 des Marknagels 1 wird als Referenz für die Position der mediolateralen Verriegelungslöcher 4 verwendet. Die Justierung der Zielschiene 8 in der anteroposterioren Ebene geschieht durch Bohren einer kleinen Kontaktöffnung in der Knochenrinde anterior zur Fusspartie 17 des implantierten Marknagels 1. In der kraniokaudalen Ebene wird die Position der Kontaktöffnung genau definiert über einen Abstandgeberbügel 12 der an der Bohrlehre 24 befestigt ist. In der mediolateralen Ebene wird die Kontaktöffnung nur grob plaziert; der Schlitten 11 am freien Ende des Abstandgeberbügels 12 erlaubt eine gewisse mediolaterale Verschiebung der Kontaktöffnung angepasst an die Anatomie des Patienten. Durch diese Kontaktöffnung wird ein spezieller L-förmiger Abstandgeber 13 geschoben und auf die anteriore Kante der Fusspartie 17 des Marknagels 1 aufgesetzt. Der L-förmige Abstandgeber 13 ermöglicht den Kontakt auf die anteriore Kante des Marknagels 1 aus einer dezentralen Position. Nach Einbringen des Abstandgebers 13 wird dieser mit der Konstruktion Schlitten 11 <=> Abstandgeberbügel 12 <=> Zielschiene 8 <=> Kupplungsmittel 10 <=> proximaler Zielbügel 2 <=> Marknagel 1 verbunden. Damit fluchten die Führungsbohrungen 23 mit den mediolateralen Verriegelungslöchern 4 und die Bohrungen können nun plaziert werden.

### C) Justierung des Zielbügels 15 in der mediolateralen Ebene auf die Position des Verriegelungsloches 3

Um die Position der Bohrung auf das anteroposteriore Verriegelungsloch 3 zu definieren, wird ein unbesetztes mediolaterales Verriegelungsloch 4 des Marknagels 1 als Referenz genommen. Der Zielbügel 15 - gekoppelt mit einem verspreizbaren Kontaktgeber 14 - wird in ein unbesetztes, gebohrtes mediolaterales Verriegelungsloch 4 durch eine der Führungsbohrungen 23 der an der Zielschiene 8 befestigten Bohrlehre 24 eingeschoben. Wenn die geschlitzte Spitze des Kontaktgebers 14 bis zum Anschlag in das Verriegelungsloch 4 des Marknagels 1 eingeführt worden ist, ist eine Spreizung der Spitze mit einem Spreizdorn 32 möglich; letzterer kann in diesem Fall bis zum Anschlag geschoben werden. Hiermit ist die Position der Führungsbohrungen 33 in der mediolateralen Ebene definiert. Für die Definition der Führungsbohrungen 33 in der kraniokaudalen Ebene müssen dieselben durch Schwenken des Zielbügels 15 mit dem Führungsschlitz 25 des Schlittens 11 in Flucht gebracht werden. Durch das Einsetzen einer passenden Bohrbüchse durch eine Führungsbohrung 33 des Zielbügels 15 und den Führungsschlitz 25 fluchtet diese Führungsbohrung 33 mit dem anterioposterioren Verriegelungsloch 3 und die Bohrung kann plaziert werden. Nun können alle Verriegelungslöcher 3,4 des Marknagels 1 mit Verriegelungsbolzen 9 besetzt werden und der Marknagel 1 ist somit in 2 verschiedenen Ebenen rotationsstabil verriegelt.

## Patentansprüche

1. Zielgerät zum Positionieren und Einbringen von osteosynthetischen Befestigungselementen (9), insbesondere von Verriegelungsschrauben oder Bolzen in die Fusspartie (17) eines in einen Knochen (36) eingebrachten Marknagels (1), das folgende Elemente umfasst:
A) einen Zielbügel (2) mit dem lösbar das Kopfende (30) eines Marknagels (1) verbunden werden kann;
B) ein Kupplungsmittel (10) das mit dem Zielbügel (2) verbunden werden kann;
C) eine longitudinale Zielschiene (8), deren erster Abschnitt (19) im Kupplungsmittel (10) verschiebbar ist, mit mindestens einer Führungsbohrung (23) im zweiten Abschnitt (20); und
D) eine am zweiten Abschnitt (20) der Zielschiene (8) fixierbare erste Abstandgebereinheit (11,12,13) mit einem Abstandgeber (13), dessen Auflagefläche auf die Oberfläche eines Marknagels (1) in dessen Fusspartie anlegbar ist;
**dadurch gekennzeichnet, dass**
E) die Zielschiene (8) an ihrem ersten Abschnitt (19) im Kupplungsmittel (10) in der Längsachse der Zielschiene (8) verschiebbar und stufenlos fixierbar und gegenüber dem Zielbügel (2) schwenkbar gelagert ist; und
F) die erste Abstandgebereinheit (11,12,13) eine Verschiebung der Auflagefläche des Abstandgebers (13) quer zur Zielschiene (8) zulässt.

2. Zielgerät nach Anspruch 1, dadurch gekennzeichnet, dass der Abstandgeber (13) L-förmig, T-förmig oder schneidenförmig ausgebildet ist.

3. Zielgerät nach Anspruch 2, dadurch gekennzeichnet, dass der kurze Schenkel des L oder T, oder die Länge der Schneide, zwischen 2 und 40 mm, vorzugsweise zwischen 8 und 20 mm beträgt.

4. Zielgerät nach einem der Ansprüche 1 - 3, dadurch gekennzeichnet, dass die zur Kontaktierung eines Marknagels (1) bestimmte Partie des Abstandgebers (13) eine Strukturierung, vorzugsweise in Form einer Verzahnung aufweist.

5. Zielgerät nach einem der Ansprüche 1 - 4, dadurch gekennzeichnet, dass über Elektrizitätsfluss eine visuelle oder sonore Rückkoppelung über den Kontakt zwischen dem Abstandgeber (13) und einem Marknagel (1) ermöglicht wird.

6. Zielgerät nach einem der Ansprüche 1- 5, dadurch gekennzeichnet, dass die erste Abstandgebereinheit (11, 12, 13) mit dem Abstandgeberbügel (12) und mit dem in einer senkrecht zur Zielschiene (8) verlaufenden Achse (27) verschiebbaren Schlitten (11) versehen ist.

7. Zielgerät nach Anspruch 6, dadurch gekennzeichnet, dass der Schlitten (11) mindestens einen Führungskanal (28) aufweist, der zum Positionieren und zur Aufnahme des Abstandgebers (13) dient.

8. Zielgerät nach einem der Ansprüche 1 - 7, dadurch gekennzeichnet, dass das Kupplungsmittel (10) am ersten Abschnitt (19) der Zielschiene (8) derart ausgebildet ist, dass eine Abwinkelung der Zielschiene (8) gegenüber dem Zielbügel (2) möglich ist.

9. Zielgerät nach Anspruch 8, dadurch gekennzeichnet, dass die Abwinkelung um eine im wesentlichen senkrecht zu der Zielschiene (8) und senkrecht zur Längsachse des Abstandgebers (13) verlaufende Gelenkachse (7) erfolgt, vorzugsweise in Form eines Drehgelenkes.

10. Zielgerät nach einem der Ansprüche 1 - 9, dadurch gekennzeichnet, dass zusätzlich eine zweite Abstandgebereinheit (14,15) vorgesehen ist, mit einem Zielbügel (15), der einerseits mit einem Kontaktgeber (14) versehen ist, der auf eine Verriegelungsbohrung (4) in der Fusspartie (17) eines Marknagels (1) Bezug nimmt, und andererseits Führungsbohrungen (33) aufweist, die zum Positionieren und Einbringen von osteosynthetischen Befestigungselementen (9) in die Fusspartie (17) eines in einen Knochen (36) eingebrachten Marknagels (1) dienen.

11. Zielgerät nach Anspruch 10, dadurch gekennzeichnet, dass die zentralen Achsen der Führungsbohrungen (33) des Zielbügels (15) parallel zur Längsachse des Abstandgebers (13) ausrichtbar sind - ohne dabei die Flucht der zentralen Achse des Schaftes (39) des Zielbügels (15) mit der zentralen Achse einer der Führungsbohrungen zu verlassen - und dabei die Achse (26) in einer parallel zur Zielschiene (8) und parallel zum Abstandgeber (13) verlaufenden Ebene ausgerichtet sein kann.

12. Zielgerät nach Anspruch 10 oder 11, dadurch gekennzeichnet, dass der Kontaktgeber (14) eine Kontaktverbindung mit einer Verriegelungsbohrung (4) in der Fusspartie (17) eines Marknagels (1) eingehen kann.

13. Zielgerät nach Anspruch 12, dadurch gekennzeichnet, dass der Kontaktgeber (14) ein verspreizbares, ein- oder mehrfach geschlitztes Ende aufweist.

14. Zielgerät nach einem der Ansprüche 12 oder 13, dadurch gekennzeichnet, dass der Kontaktgeber (14) Abstandgeberflächen (44) aufweist, die im gespreizten Zustand des Kontaktgebers (14) ein Verschieben des Kontaktgebers (14) in einer Verriegelungsbohrung (4) eines Marknagels (1) verhindern.

15. Zielgerät nach einem der Ansprüche 12 bis 14, dadurch gekennzeichnet, dass der Kontaktgeber (14) aus zwei ineinander verschiebbaren Abstandgebern besteht, einer mit Kontakt auf der einen Seite, der andere mit Kontakt auf der anderen Seite eines Verriegelungsloches (4) eines Marknagels (1).

16. Zielgerät nach einem der Ansprüche 12 - 15, dadurch gekennzeichnet, dass über Elektrizitätsfluss eine visuelle oder sonore Rückkoppelung über den Kontakt zwischen dem Kontaktgeber (14) und einem Marknagel (1) ermöglicht wird.

17. Zielgerät nach einem der Ansprüche 1 - 16, dadurch gekennzeichnet, dass der Zielbügel (2) mittels eines Schraubenverschlusses (29) am Kopfende (30) eines Marknagels (1) befestigbar ist.

18. Zielgerät nach Anspruch 17, dadurch gekennzeichnet, dass die Zielschiene (8) um eine als Drehgelenk dienende Gelenkachse (7), welche senkrecht zur Zielschiene (8) verläuft und die verlängerte Achse des Schraubenverschlusses (29) schneidet, schwenkbar ist.

## Claims

1. Targeting apparatus for positioning and inserting osteosynthetic fasteners (9), particularly of locking screws or bolts into the base part (17) of a intramedullary nail (1) inserted into a bone (36) comprising the following elements:
A) an alignment bracket (2) whereto the top end (30) of an intramedullary nail (1) may be detachably connected;
B) a coupling unit (10) which may be connected to the alignment bracket (2);
C) a longitudinal alignment bar (8) having a first section (19) which is displaceable in the coupling unit (10), with at least one guide hole (23) in the second section (20); and
D) a first spacing-sensor assembly (11;12;13) attachable to the second section (20) of the alignment bar (8) and provided with a spacing sensor (13) having a contact surface for placement on the surface of the base part (17) of an intramedullary nail (1),
characterized in that
E) the alignment bar (8) is displaceable in the longitudinal axis of the alignment bar (8) and lockable with its first section (19) within the coupling unit (10) and pivotable relative to the alignment bracket (2); and
F) the first spacing-sensor assembly (11;12;13) permits the contact surface of the spacing sensor (13) to shift transversely to the alignment bar (8).

2. Targeting apparatus according to claim 1, characterized in that the spacing sensor (13) has an L, T or edge shape.

3. Targeting apparatus according to claim 2, characterized in that the short leg of the L or T or the length of the edge having a length of 2 and 40 mm, preferably between 8 and 20 mm.

4. Targeting apparatus according to one of the claims 1 to 3, characterized in that section of the spacing sensor (13) which is determined for contacting the intramedullary nail (1) is provided with a structure, preferably in the form of a tooth system.

5. Targeting apparatus according to one of the claims 1 to 4, characterized in that an electricity flow permits a optical or audible feedback indicating contact between the spacing sensor (13) and an intramedullary nail (1).

6. Targeting apparatus according to one of the claims 1 to 5, characterized in that the first spacing-sensor assembly (11;12;13) includes a slide (11) which is movable along an axis (27) extending perpendicular to the alignment bar (8).

7. Targeting apparatus according to claim 6, characterized in that the slide (11) includes at least one guide channel (28) serving to permit positioning and accommodation of the spacing sensor (13).

8. Targeting apparatus according to one of the claims 1 to 7, characterized in that the coupling unit (10) at the first section (19) of the alignment bar (8) is configured to allow angling of the alignment bar (8) relative to the alignment bracket (2).

9. Targeting apparatus according to claim 8, characterized in that the change of angle takes place around a pin (7) which extends essentially perpendicular to the alignment bar (8) and perpendicular to the longitudinal axis of the spacing-sensor assembly (13) and is preferably as a pivoting joint.

10. Targeting apparatus according to one of the claims 1 to 9, characterized in that it further comprises a second spacing sensor assembly (14;15) with an alignment bridge (15) which is provided on one side with a contactor (14) relating to a locking hole (4) in the base part (17) of the intramedullary nail (1), and on the other side with guide holes (33) that serve to the positioning and insertion of osteosynthetic fasteners (9) into the base part (17) of an intramedullary nail (1) inserted in a bone (36).

11. Targeting apparatus according to claim 10, characterized in that the central axes of the guide holes (33) of the alignment bridge (15) are adjustable parallel to the longitudinal axis of the spacing sensor (13) - without leaving the alignment of the central axis of the shaft (39) of the alignment bridge (15) with the central axes of one of the guide holes - and that the axis (26) may be aligned in a plane extending parallel to he alignment bar (8) and parallel to the spacing sensor (13).

12. Targeting apparatus according to claim 10 or 11, characterized in that the contactor (14) may contact a locking hole (4) in the base part (17) of an intramedullary nail (1).

13. Targeting apparatus according to claim 12, characterized in that the contactor (14) is provided with an expandable single- or multi-slot tip.

14. Targeting apparatus according to claim 12 or 13, characterized in that the contactor (14) is provided with spacing-sensor surfaces (44) which in the expanded state of the contactor (14) prevent shifting of the contactor (14) in the locking hole (4) of the intramedullary nail (1).

15. Targeting apparatus according to one of the claims 12 to 14, characterized in that the contactor (14) consists of two interlocked mutually telescopable spacing sensors, one of which makes contact on one side of the locking hole (4) of the intramedullary nail (1) while the other makes contact on the other side of the locking hole (4) of the intramedullary nail (1).

16. Targeting apparatus according to one of the claims 12 to 15, characterized in that via electrical flow permits an optical or audible feedback between the contactor (14) and an intramedullary nail (1).

17. Targeting apparatus according to one of the claims 1 to 16, characterized in that the alignment bracket (2) is fastenable at the top end (30) of a intramedullary nail (1) by means of a screw-type fastener (29).

18. Targeting apparatus according to claim 17, characterized in that the alignment bar (8) is rotatable around a pivot pin (7) which serves as an axis of rotation and extends perpendicular to the alignment bar (8) and intersects an axis which extends from the screw-type fastener (29).

## Revendications

1. Appareil de visée pour le positionnement et l'insertion d'éléments de fixation d'ostéosynthèse (9), en particulier de vis de verrouillage ou de boulons, dans la partie de pied (17) d'un clou médullaire
(1) inséré dans un os (36), qui comporte les éléments suivants :
A) un étrier de visée (2) avec lequel l'extrémité de tête (30) d'un clou médullaire (1) peut être relié de manière libérable;
B) un moyen d'accouplement (10) qui peut être relié à l'étrier de visée (2);
C) un rail longitudinal de visée (8) dont une premier partie (19) peut coulisser dans le moyen d'accouplement (10), et avec au moins un alésage de guidage (23) dans la deuxième partie (20); et
D) une première unité d'écartement (11,12,13) qui peut être fixée sur la deuxième partie (20) du rail de visée (8), et qui présente un écarteur (13) dont la surface de pose peut être placée à la surface d'un clou médullaire (1) dans la partie de pied de ce dernier;
caractérisé en ce que
E) la première partie (19) du rail de visée (8) peut coulisser dans le moyen d'accouplement (10) suivant l'axe longitudinal du rail de visée (8), peut être immobilisée de manière continue et est montée de manière à pouvoir pivoter par rapport à l'étrier de visée (2); et
F) la première unité d'écartement (11, 12, 13) permet un coulissement de la surface de pose de l'écarteur (13) transversalement par rapport au rail de visée (8).

2. Appareil de visée selon la revendication 1, caractérisé en ce que l'écarteur (13) est configuré en forme de L, en forme de T ou en forme de lame.

3. Appareil de visée selon la revendication 2, caractérisé en ce que la courte branche du L ou du T ou la longueur de la lame est comprise entre 2 et 40 mm, de préférence entre 8 et 20 mm.

4. Appareil de visée selon l'une des revendications 1 à 3, caractérisé en ce que la partie de l'écarteur (13) destinée à entrer en contact avec un clou médullaire (1) présente une structuration, de préférence sous la forme d'une dentelure.

5. Appareil de visée selon l'une des revendications 1 à 4,
caractérisé en ce que l'écoulement d'un courant électrique permet une rétroaction visuelle ou sonore concernant le contact entre l'écarteur (13) et un clou médullaire (1).

6. Appareil de visée selon l'une des revendications 1 à 5, caractérisé en ce que la première unité d'écartement (11, 12, 13) est dotée de l'étrier écarteur (12) et du coulisseau (11) qui peut coulisser suivant un axe (27) qui s'étend perpendiculairement au rail de visée (8).

7. Appareil de visée selon la revendication 6, caractérisé en ce que le coulisseau (11) présente au moins un canal de guidage (28) qui sert au positionnement et à la réception de l'écarteur (13).

8. Appareil de visée selon l'une des revendications 1 à 7 caractérisé en ce que le moyen d'accouplement (10) prévu sur la première partie (19) du rail de visée (8) est configuré de manière à permettre au rail de visée (8) d'être placé en position oblique par rapport à l'étrier de visée (2).

9. Appareil de visée selon la revendication 8, caractérisé en ce que l'obliquité s'effectue autour d'un axe d'articulation (7), de préférence sous la forme d'une articulation en rotation, qui s'étend essentiellement à la perpendiculaire du rail de visée (8) et à la perpendiculaire de l'axe longitudinal de l'écarteur (13).

10. Appareil de visée selon l'une des revendications 1 à 9, caractérisé en ce qu'une deuxième unité d'écartement (14, 15) est prévue en supplément, et comporte un étrier de visée (15) qui est d'une part doté d'une sonde de contact (14) qui se réfère à un alésage de verrouillage (4) prévu dans la partie de pied (17) d'un clou médullaire (1) et qui d'autre part présente des alésages de guidage (33) qui servent au positionnement et à l'insertion d'éléments (9) de fixation d'ostéosynthèse dans la partie de pied (17) d'un clou médullaire (1) installé dans un os (36).

11. Appareil de visée selon la revendication 10, caractérisé en ce que les axes centraux des alésages de guidage (33) de l'étrier de visée (15) peuvent être orientés parallèlement à l'axe longitudinal de l'écarteur (13) sans perdre à cette occasion l'alignement de l'axe central de la tige (39) de l'étrier de visée (15) sur l'axe central de l'un des alésages de guidage, et ainsi, l'axe (26) peut être orienté dans un plan qui s'étend parallèlement au rail de visée (8) et parallèlement à l'écarteur (13).

12. Appareil de visée selon les revendications 10 ou 11, caractérisé en ce que la sonde de contact (14) peut entrer en liaison de contact avec un alésage de verrouillage (4) prévu dans la partie de pied (17) d'un clou médullaire (1).

13. Appareil de visée selon la revendication 12, caractérisé en ce que la sonde de contact (14) présente une extrémité fendue une ou plusieurs fois et apte à être déployée.

14. Appareil de visée selon l'une des revendications 12 ou 13, caractérisé en ce que la sonde de contact (14) présente des surfaces d'écartement (44) qui, lorsque la sonde de contact (14) est à l'état déployé, empêche que la sonde de contact (14) coulisse dans un alésage de verrouillage (4) d'un clou médullaire (1).

15. Appareil de visée selon l'une des revendications 12 à 14 caractérisé en ce que la sonde de contact (14) est constituée de deux écarteurs pouvant coulisser l'un dans l'autre, l'un en contact avec un côté et l'autre en contact avec l'autre côté d'un trou de verrouillage (4) d'un clou médullaire (1).

16. Appareil de visée selon l'une des revendications 12 à 15, caractérisé en ce que l'écoulement d'un courant électrique permet une rétroaction visuelle ou sonore concernant le contact entre la sonde de contact (14) et un clou médullaire (1).

17. Appareil de visée selon l'une des revendications 1 à 16, caractérisé en ce que l'étrier de visée (2) peut être fixé à l'extrémité de tête (30) d'un clou médullaire (1) au moyen d'une fermeture à vis (29).

18. Appareil de visée selon la revendication 17, caractérisé en ce que le rail de visée (8) peut pivoter autour d'un axe d'articulation (7) qui sert d'articulation en rotation, qui s'étend perpendiculairement au rail de visée (8) et qui coupe la prolongation de l'axe de la fermeture à vis (29).
